# EUROPEAN PATENT APPLICATION

(11) **EP 4 694 037 A1**
(43) Date of publication of application: **11.02.2026**
(21) Application number: 25194126.6
(22) Date of filing: 05.08.2025
(51) Int. Cl.: H04L 9/40, G16H 40/00

(54) **STATELESS, SCALABLE AND LIGHTWEIGHT COMMUNICATION PROTOCOL**

(30) Priority: 07.08.2024 US 202463680477 P; 28.07.2025 US 202519282278
(71) Applicant: Medtronic MiniMed, Inc., Northridge, CA 91325-1219 (US)
(72) Inventor: BERIAN, Jesus, Northridge, 91325 (US); ROSALES, Miguel F., Northridge, 91325 (US); SERRANO, Eduardo Javier, Northridge, 91325 (US); NGUYEN, Vincent K., Northridge, 91325 (US)
(74) Representative: Zimmermann & Partner Patentanwälte mbB

(57) **Abstract**

A stateless, scalable, and lightweight communication protocol may support multiple message formats that allow messages to be processed independently and with varying levels of security. When transmitting a message over a communication channel using the communication protocol, an electronic device may determine a message format for the message. The message format can be one of a plurality of message formats available for messages communicated over the communication channel. Each message format is associated with a corresponding level of security. At least one of the message formats may require message encryption. The electronic device can generate the message according to the determined format and then transmit the message to a second electronic device over the communication channel.

## Description

### CROSS-REFERENCE TO RELATED APPLICATION

This application claims the benefit of and priority to U.S. Provisional Application No. 63/680,477, filed August 7, 2024, entitled "STATELESS, SCALABLE AND LIGHTWEIGHT COMMUNICATION PROTOCOL," which is assigned to the assignee hereof and is hereby incorporated by reference in its entirety for all purposes.

### TECHNICAL FIELD

The present disclosure relates generally to secured electronic communication, in particular to communications using a communication protocol that supports encryption of messages. Aspects of the disclosure are directed to communication between devices that are authenticated through a public key infrastructure including, in some instances, medical devices.

### BACKGROUND

Electronic communications are often encrypted for security. Many cryptographic algorithms rely on shared secrets (e.g., encryption keys and decryption keys). Encryption may involve symmetric key cryptography or asymmetric key cryptography. Symmetric key cryptography uses the same key to both encrypt and decrypt data. Asymmetric key cryptography (also known as public key cryptography) uses a combination of public and private keys to encrypt and decrypt data. For example, a first electronic device may use its own private key to encrypt a message for transmission to a second electronic device, and the second electronic device may decrypt the message using the first electronic device's public key. The electronic devices can share their public keys with each other while keeping their own private keys secret.

The choice between symmetric key cryptography and public key cryptography can depend on a variety of factors, such as operating environment, performance requirements or constraints, etc. Some situations may warrant use of one type of cryptography over another type. Additionally, some situations may benefit from having no encryption whatsoever. Systems that are configured to apply encryption uniformly across communications tend to be less adaptable to different situations.

### SUMMARY

The present disclosure relates generally to secured electronic communication, in particular to communications using a communication protocol that supports encryption of messages. More specifically, techniques disclosed herein relate to a scalable, lightweight communication protocol that can provide varying levels of security without mandating the use of encryption for every message communicated over a communication channel established between communication endpoints. In some embodiments, a communication protocol may also be a stateless protocol that supports individual processing of messages (e.g., receiver-side decoding and/or decryption) in a manner that does not require maintaining information about connection state across different messages sent between communication endpoints. With a stateless protocol, messages can be processed out-of-order and independently of each other. These and other benefits are described in more detail below with respect to various example embodiments.

A system may include one or more processors and one or more processor-readable storage media storing instructions which, when executed by the one or more processors, cause performance of determining a first message format for a first message to be transmitted to a second electronic device over a communication channel between the first electronic device and the second electronic device. The first message is part of a sequence of messages communicated over the communication channel. The first message format is one of a plurality of message formats available for messages communicated over the communication channel. Each message format in the plurality of message formats is associated with a corresponding level of security. At least one message format in the plurality of message formats requires a message to be encrypted. The first message format allows the first message to be processed independently of other messages in the sequence of messages. The instructions further cause performance of generating the first message according to the first message format and transmitting the first message to the second electronic device over the communication channel.

A processor-implemented method may include determining, by one or more processors of a first electronic device, a first message format for a first message to be transmitted to a second electronic device over a communication channel between the first electronic device and the second electronic device. The first message is part of a sequence of messages communicated over the communication channel. The first message format is one of a plurality of message formats available for messages communicated over the communication channel. Each message format in the plurality of message formats is associated with a corresponding level of security. At least one message format in the plurality of message formats requires a message to be encrypted. The first message format allows the first message to be processed independently of other messages in the sequence of messages. The method may further include generating the first message according to the first message format and transmitting the first message to the second electronic device over the communication channel.

One or more non-transitory processor-readable storage media may store instructions which, when executed by one or more processors of a first electronic device, cause performance of determining a first message format for a first message to be transmitted to a second electronic device over a communication channel between the first electronic device and the second electronic device. The first message is part of a sequence of messages communicated over the communication channel. The first message format is one of a plurality of message formats available for messages communicated over the communication channel. Each message format in the plurality of message formats is associated with a corresponding level of security. At least one message format in the plurality of message formats requires a message to be encrypted. The first message format allows the first message to be processed independently of other messages in the sequence of messages. The instructions further cause performance of generating the first message according to the first message format and transmitting the first message to the second electronic device over the communication channel.

Further disclosed herein is a stateless, scalable, and lightweight communication protocol that may support multiple message formats that allow messages to be processed independently and with varying levels of security. When transmitting a message over a communication channel using the communication protocol, an electronic device may determine a message format for the message. The message format can be one of a plurality of message formats available for messages communicated over the communication channel. Each message format is associated with a corresponding level of security. At least one of the message formats may require message encryption. The electronic device can generate the message according to the determined format and then transmit the message to a second electronic device over the communication channel.

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

### BRIEF DESCRIPTION OF THE DRAWINGS

Illustrative embodiments are described in detail below with reference to the following figures.
FIG. 1 illustrates an example of a system according to certain embodiments.
FIG. 2 illustrates an example of messages being communicated in accordance with a stateful communication protocol.
FIG. 3 is a flow diagram illustrating an example of messages that have different security levels and are communicated out-of-order, according to certain embodiments.
FIG. 4A is a high-level conceptual illustration of a message and its contents, according to certain embodiments.
FIG. 4B illustrates an example of security artifacts that may be used by a communication endpoint, according to certain embodiments.
FIG. 4C illustrates an example of different message formats that may be available when communicating using a stateless and scalable communication protocol, according to certain embodiments.
FIG. 5 is a flow diagram of a method for communicating over a communication channel, according to certain embodiments.
FIG. 6 is a flow diagram of a method for communicating over a communication channel, according to certain embodiments.
FIG. 7 illustrates an example therapy delivery system in accordance with certain aspects of the present disclosure.
FIG. 8 illustrates an example of a delivery device, which may implement some of the examples disclosed herein.
FIG. 9 is a simplified block diagram of an example electronic device that may implement some of the examples disclosed herein.

### DETAILED DESCRIPTION

The present disclosure relates generally to secured electronic communication, in particular to communications using a communication protocol that supports encryption of messages. More specifically, techniques disclosed herein relate to a scalable, lightweight communication protocol that can provide varying levels of security without mandating the use of encryption for every message communicated over a communication channel established between communication endpoints. For example, one security level may involve encryption, another security level may involve a different (e.g., more secure) form of encryption plus digital signature, and yet another security level may involve digital signature without encryption. In some embodiments, a communication protocol may also be a stateless protocol that supports individual processing of messages (e.g., receiver-side decoding and/or decryption) in a manner that does not require maintaining information about connection state across different messages sent between endpoints. With a stateless protocol, messages can be processed out-of-order and independently of each other. This is because each message is self-contained and expected to include enough information for the receiver to be able to process (e.g., decode and/or decrypt) the message without referring to information from earlier messages. Thus, a message can be processed even if the message is received out of order or another message is lost.

In some embodiments, the communication protocol may use security mechanisms provided by a public key infrastructure (PKI). A PKI generally includes one or more computer systems responsible for implementing policies and procedures related to digital certificates and public-key encryption. Digital certificates can be used for device authentication. For example, a digital certificate issued to an electronic device may include the device's public key together with information identifying the device and a digital signature of an entity that issued the digital certificate. Thus, the communication protocol may leverage a PKI for security-related functions such as identity management, authentication, and authorization.

Additionally, the communication protocol may be compatible with other (e.g., lower level) protocols that are potentially used for electronic communication. For example, the communication protocol may be agnostic to the implementation details of the transport layer (layer 4 of the Open Systems Interconnection (OSI) model). Thus, the communication protocol may function equally as well irrespective of whether the system in which communications take place is configured to transport messages using Transmission Control Protocol (TCP), User Datagram Protocol (UDP), or some other transport layer protocol.

For purposes of illustration and explanation, various examples described in this disclosure are related to medical devices, including insulin or fluid delivery devices. However, the techniques disclosed herein can be applied to other environments in which secured communications are conducted between electronic devices. Illustrative examples of systems, devices, and methods embodying aspects of these techniques are described with reference to the accompanying drawings. The embodiments can be combined in different ways and modified/adapted as appropriate depending on application.

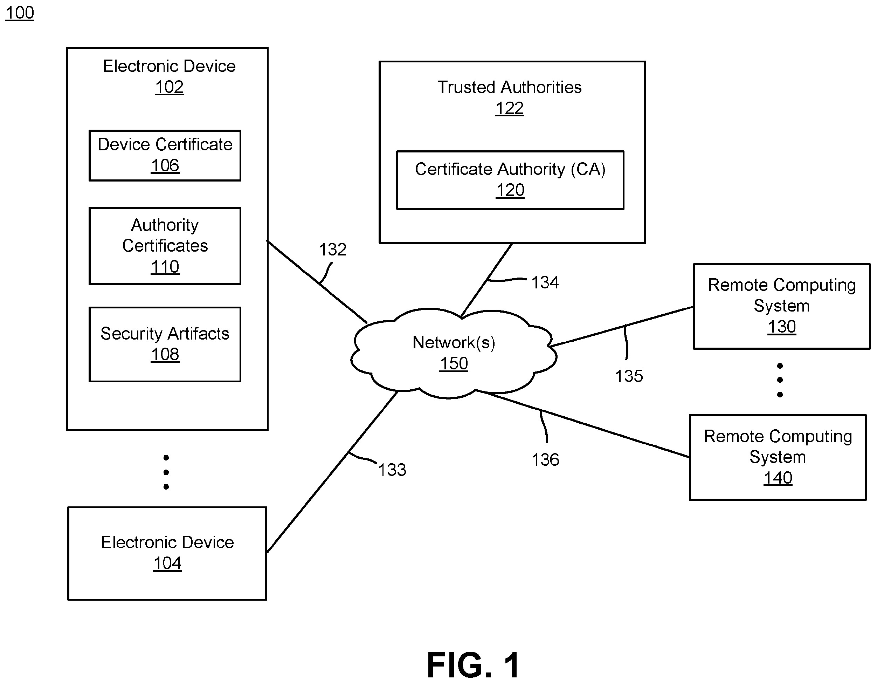

**FIG.** 1 shows an example of a system 100 according to certain embodiments. The system 100 includes components that implement a public key infrastructure (PKI). FIG. 1 is merely an example of how the techniques disclosed herein may be integrated into a system in which authentication (e.g., device authentication and/or user authentication) is performed in connection with electronic communications. In some embodiments, the system 100 may be configured to provide authentication and other security-related functions without a PKI.

In the example of FIG. 1, the components responsible for providing the PKI include one or more trusted authorities 122, e.g., a certificate authority (CA) 120. The authorities 122 are trusted entities whose responsibilities may include, among other things, issuing device certificates for electronic devices that participate in secured communications within the system 100. The trusted authorities 122 can be implemented in hardware, software, or a combination of hardware and software. As such, the trusted authorities 122 may correspond to logical entities that physically reside in a centralized location (e.g., a server computer) or are distributed across different computer systems.

CA 120 may be responsible for issuing digital certificates to non-authority entities. For example, the CA 120 may be configured to issue device certificates to a first electronic device 102 and a second electronic device 104. The CA 120 need not be a root of trust. In some embodiments, the trusted authorities 122 may be arranged in a trust hierarchy, with a root CA (not shown) being the root of trust. Specifically, the root CA may have the highest level of trust in the PKI and may be issued (or self-generate) a root certificate representing the identity of the root CA. The root certificate may be valid indefinitely (e.g., with no expiration date) or have a comparatively long validity period relative to certificates issued to other entities. In embodiments featuring a root CA, the root CA may be configured to issue a CA certificate to the CA 120. The CA certificate represents the identity of the CA 120 and may give the CA 120 the ability to issue certificates for non-authority entities.

In some instances, the CA 120 may issue a certificate in response to a signature request. For example, an electronic device may send the CA 120 a self-generated certificate containing information identifying the electronic device (e.g., a device name or ID). The self-generated certificate may also include a public key associated with the electronic device (e.g., the public portion of a public-private key pair generated by the electronic device). The CA 120 may issue the electronic device a device certificate corresponding to a signed version of the self-generated certificate. The device certificate would include a digital signature proving the authenticity of the device certificate, and therefore the identity of the bearer of the device certificate. In some embodiments, device certificates may be formatted in accordance with the X.509 certificate standard. Device certificates can be issued as part of enrolling electronic devices into the PKI of the system 100. Electronic devices may also periodically request new device certificates (e.g., when an existing device certificate has expired or is about to expire). The CA 120 may verify the identity of a device requesting a device certificate (e.g., by checking the contents of the self-generated certificate) as a condition for issuing the device certificate. The CA 120 may also revoke device certificates in some circumstances (e.g., in response to a security breach that exposed an electronic device's private key).

The digital signature for a device certificate can be generated in various ways, such as through computing a hash of the self-generated certificate and encrypting the hash using a private key of the CA 120. The hash can be computed using a standard hash algorithm, such as Secure Hash Algorithm 2 with 256 bits (SHA256). The device certificate can be validated by decrypting the hash using a public key of the CA 120, computing a hash of the device certificate as received by the entity performing the validation, and comparing the decrypted hash to the newly computed hash. Thus, device certificates can be used to authenticate electronic devices.

System 100 may further include one or more remote computing systems (e.g., a first remote computing system 130 and a second remote computing system 140). Each remote computing system may include one or more computing devices (e.g., a cloud server). The remote computing system(s) may be configured to communicate with electronic devices 102, 104 to provide various services to the electronic devices 102, 104. For example, the electronic device 102 may be a smartphone, and the remote computing system 130 may communicate with a software application running on the smartphone. The remote computing system 130 may be configured to process data received from the smartphone and return a result to the software application. In some instances, the processing of the data received from the smartphone may involve communicating with another computing system (e.g., retrieving data stored at the remote computing system 140).

The components in the system 100 are communicatively intercoupled through one or more networks 150. The network(s) 150 may include wired and/or wireless networks that are accessed through a plurality of communications links, such as communication links 132-136. The communications links 132-136 may each be a wired connection and/or a wireless connection. Although not shown in FIG. 1, the electronic devices 102 and 104 may also be communicatively coupled to each other through communications links. In embodiments where two devices are located in the same device housing, the communication links may include, for example, wires, cables, and/or communication buses on a printed circuit board, among other things. In embodiments where two devices are separated from each other in different device housings, the communication links may be wired and/or wireless connections.

Wired connections may include, without limitation, an Ethernet connection, a Universal Serial Bus (USB) connection, and/or another type of physical connection. Wireless connections may include, without limitation, a cellular connection, a Wi-Fi connection, a Bluetooth^{®} connection, a mesh network connection, and/or another type of connection using a wireless communication protocol. Some communication links may use direct connections, such as Bluetooth^{®} connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the above-described communication links.

As an illustrative example, the electronic devices 102, 104 may establish a communication channel for communicating with each other using a Bluetooth^{®} or near field communication (NFC) protocol. To establish the communication channel, the electronic devices 102, 104 may engage in a pairing or bonding procedure in which the electronic devices exchange certain information and then validate the exchanged information. In some embodiments, the initial setup of the communication channel may involve exchanging device certificates and/or CA certificates. For example, the electronic device 102 may include a device certificate 106 issued by the CA 120. Further, the electronic device 102 may include a CA certificate (not shown) representing the identity of the CA 120. The CA certificate may be stored locally in a memory system of the electronic device 102 and as part of a set of authority certificates 110.

The device certificate 106 may also be stored locally and can be a digital certificate that was generated by the electronic device 102 and then signed by the CA 120 before being returned to the electronic device 102. The device certificate 106 may include information identifying the electronic device 102 (e.g., a device name, a hardware address, a network address, and/or other metadata) and a public key associated with the electronic device 102. The public key may belong to a set of security artifacts 108 used by the electronic device 102. For example, the security artifacts 108 may further include a private key associated with the electronic device 102. The electronic device 102 may use the private key to encrypt certain types of messages for transmission to the electronic device 104 or some other recipient, and the messages may be decrypted using the public key.

Like the electronic device 102, the electronic device 104 may store its own device certificate and security artifacts. The device certificate of the electronic device 104 is typically issued by the CA 120 at a different time than the device certificate 106 of the electronic device 102. This is because each electronic device 102, 104 may be configured to independently request issuance of its corresponding device certificate. Further, electronic devices may be added to or removed (enrolled/unenrolled) from the system 100 over time. For example, new electronic devices joining the system may be registered with one of the trusted authorities 122 and issued device certificates at the time of registration.

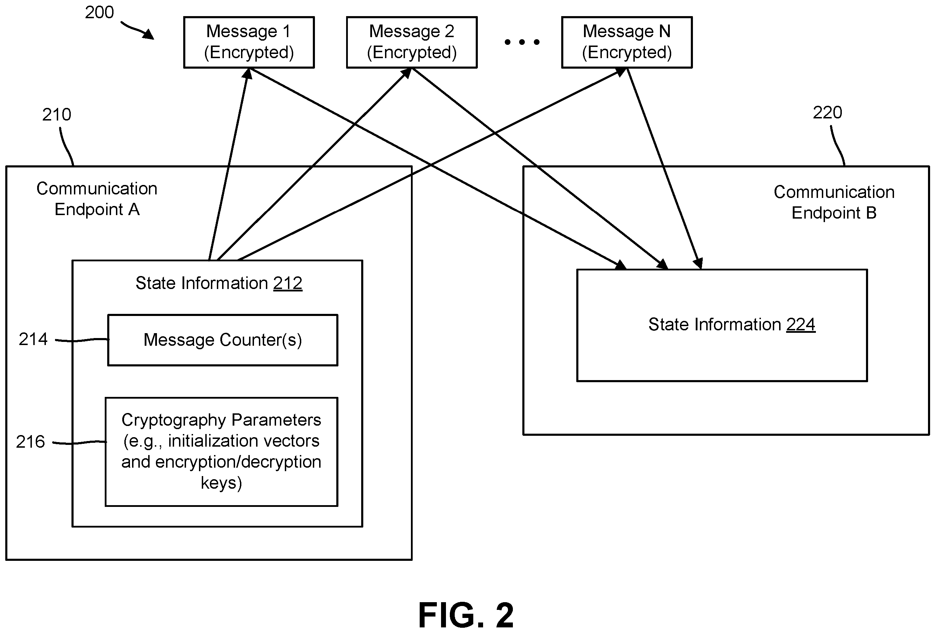

**FIG.** 2 illustrates an example of messages being communicated in accordance with a stateful (i.e., not stateless) communication protocol. For example, FIG. 2 may be representative of communications conducted in accordance with a Transport Layer Security (TLS) protocol. A TLS protocol (e.g., TLS version 1.3) generally requires that every message communicated between endpoints be encrypted. The cryptographic keys used to encrypt and decrypt the messages may be updated during a TLS session (e.g., over the course of a message sequence 200 between a first communication endpoint 210 and a second communication endpoint 220). The endpoints 210, 220 can be electronic devices. For example, the endpoint 210 may correspond to the first electronic device 102, and the endpoint 220 may correspond to the second electronic device 104. Communication endpoints can also be computing devices or systems. By way of example, the electronic devices 102, 104 may at various times communicate with the trusted authorities 122, the remote computing system 130, and/or the remote computing system 140.

For illustration purposes, FIG. 2 shows the endpoint 210 as being the sender of every message in the message sequence 200. For example, the endpoint 210 may be the source of Message 1, Message 2, and Message N, and the endpoint 220 may be destination of each of these messages. However, communication sessions are typically bi-directional, with messages being sent back and forth between endpoints.

One characteristic of a stateful communication protocol is that messages may have to be received and processed in the same order that the messages were transmitted. This is because the connection state usually changes from one message to the next, so that a current connection state is dependent on a previous connection state. As such, each endpoint may be required to maintain up-to-date state information. If the state information is outdated, an endpoint may no longer be able to process any messages it subsequently receives. Similarly, an endpoint may stop transmitting messages when its state information is outdated. Depending on the usage scenario, in-order processing and encryption-by-default can be an advantage or a disadvantage.

Accordingly, the endpoint 210 may be configured to maintain state information 212, and the endpoint 220 may be configured to maintain state information 224. Each endpoint is generally responsible for updating its own state information. The state information maintained by an endpoint may include, among other things, message counters and cryptography parameters. For example, the state information 212 of the endpoint 210 may include one or more message counters 214 and cryptography parameters 216. In some instances, the message counters 214 may include a read counter and a separate write counter. The message counter(s) 214 are updated as the endpoint 210 transmits or receives messages. For example, the endpoint 210 may increment its write counter each time it transmits a message to the endpoint 220.

Cryptography parameters 216 may include initialization vectors, encryption keys, and decryption keys. An initialization vector (sometimes referred to as a starting variable) is used as input to a cryptographic algorithm to provide an initial state. An initialization vector generally includes random or pseudorandom values to increase the security of encrypted messages. The way in which encryption keys and decryption keys are generated may be defined by protocol. Symmetric keys or asymmetric keys may be used. Like the message counter(s) 214, the cryptography parameters 215 are subject to change. For example, an encryption key used for Message 1 may be different from an encryption key used for Message 2, and the encryption key for Message 2 may be generated based on the state information 212 existing at a time of Message 1.

The state information 212 and the state information 224 are generally expected to be synchronized with each other. For example, a TLS protocol may require that an endpoint maintain separate read and write states. Thus, the read state of the endpoint 210 should match or be consistent with the write state of the endpoint 220. Likewise, the write state of the endpoint 210 should match or be consistent with the read state of the endpoint 220. When the endpoint 210 transmits Message 1, the endpoint 210 will update the state information 212 accordingly. Further, it is expected that the endpoint 220 will update its own state information 224 when receiving Message 1 to reflect the changes in the state information 212. The same applies to the other messages in the message sequence 200 (e.g., Message 2 and Message N).

Updating the state information 212, 224 requires that messages be transmitted and received in-order. This is because knowledge of the state information for an earlier message (e.g., Message 1) is usually needed to correctly determine the state information for a later message (e.g., Message 2, which is the next message after Message 1). Keeping state information up to date can consume a significant amount of computing resources (e.g., processor time and memory) relative to the resources available to the endpoints. Some devices have very limited resources. For example, an electronic device in a medical system may only have several hundred kilobytes of random-access memory (RAM) on-board. Maintaining state information in such instances may require dedicating a large portion of the memory, which could be better utilized for other purposes. In extreme cases, the electronic device may experience performance slowdowns from running out of free resources.

Further, even if updating the state information is not computationally intensive or memory intensive, the updating could consume a large amount of electrical power over time since updates are performed for each message sent or received. Many medical devices have small form factors for portability, user comfort (e.g., in the case of an implantable or wearable device), and other reasons. Consequently, power sources in such devices tend to be of limited capacity. Devoting power to maintaining state information may reduce the operating time of the device, leading to frequent recharging, battery replacement, etc. In the case of a device with a non-rechargeable and non-replaceable battery, the lifespan of such a device could be shortened significantly.

Another drawback to stateful communication protocols is that communication generally cannot continue if one or more messages fail to be received. This can occur, for example, in operating environments that are susceptible to dropped packets due to electrical noise or interference. Devices that rely on short-range wireless communication (e.g., using Bluetooth^{®} Low Energy (BLE) or NFC) are especially prone to packet loss. For these and other reasons, it may be beneficial to employ a stateless and lightweight communication protocol in situations where there is no need to encrypt every message. As discussed in further detail below, such a protocol may also be scalable to support a range of security levels with varying degrees of data protection (e.g., at least one option besides encryption and no encryption).

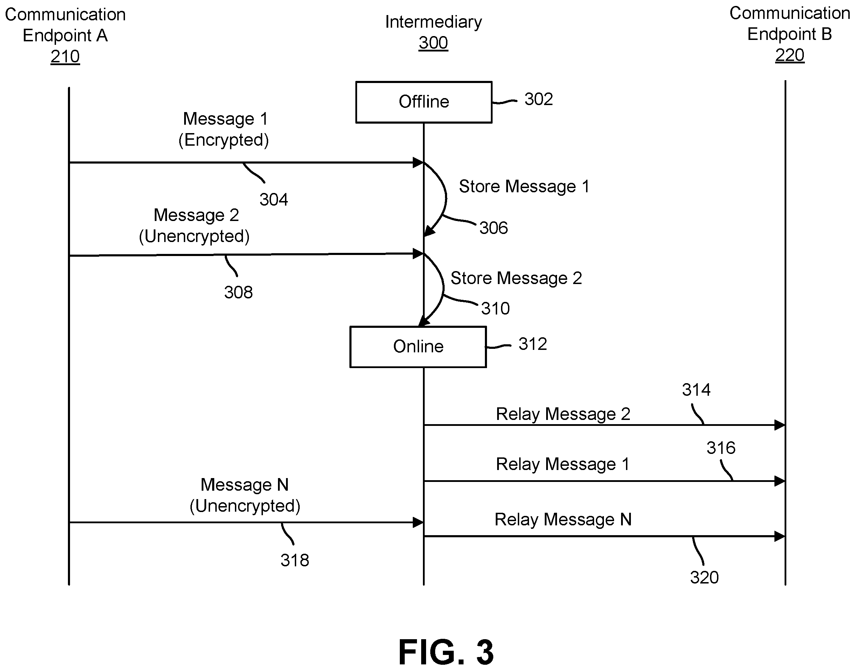

**FIG. 3** is a flow diagram illustrating an example of messages that have different security levels and are communicated out-of-order, according to certain embodiments. FIG. 3 is also an example of a more complex communication scenario involving an intermediary 300, unlike the direct point-to-point scenario depicted in FIG. 2. Suppose that among the messages shown in FIG. 2, only Message 1 needs to be encrypted for security reasons. For example, the endpoint 210 could be a glucose monitor device that periodically sends data to a cloud computing system (e.g., the endpoint 220) through the intermediary 300. The intermediary 300 could be a smartphone running a software application configured to communicate with both endpoints 210, 220. The endpoint 210 may be configured (e.g., programmed in firmware) to encrypt medical data or other types of sensitive data while permitting non-sensitive data to be sent unencrypted.

At 302, the intermediary 300 is initially offline. For example, the smartphone may be in an environment with no cellular reception or Wi-Fi to connect to the cloud computing system over Internet. However, the smartphone may still be able to communicate with the glucose monitor device through a wireless connection (e.g., using BLE).

At 304, the endpoint 210 transmits Message 1 to the intermediary 300.

At 306, since the intermediary 300 has no network connection to the endpoint 220 currently, the intermediary 300 stores Message 1 in a local cache. In some instances, the local cache may be configured as a last-in, first-out (LIFO) buffer.

At 308, the endpoint 210 transmits Message 2 to the intermediary 300.

At 310, the intermediary 300 stores Message 2 in the local cache.

At 312, the intermediary 300 comes back online.

At 314, the intermediary relays Message 2 to the endpoint 220 since Message 2 was the last message to be written to the local cache.

At 316, the intermediary relays Message 1 to the endpoint 220. At this time, all messages from the endpoint 210 have been communicated to the endpoint 220. However, Message 1 arrives at the endpoint 220 out of order relative to Message 2.

At 318, the endpoint 210 transmits Message N to the intermediary 300.

At 320, the intermediary 300 relays Message N without caching the message since the intermediary 300 is online.

FIG. 3 is just one example of a scenario in which messages are communicated out of order. There are other reasons why messages could become out of order, for instance, due to retransmission of lost packets. Based on the above discussion of FIGS. 2 and 3, it should be apparent that out-of-order processing is not possible with a stateful communication protocol. Thus, the endpoint 220 in FIG. 3 may be unable to decode Message 1 and Message 2 and possibly even Message N. Further, when using a stateful protocol, the endpoint 210 may be forced to encrypt all three messages before sending each of them to the intermediary 300.

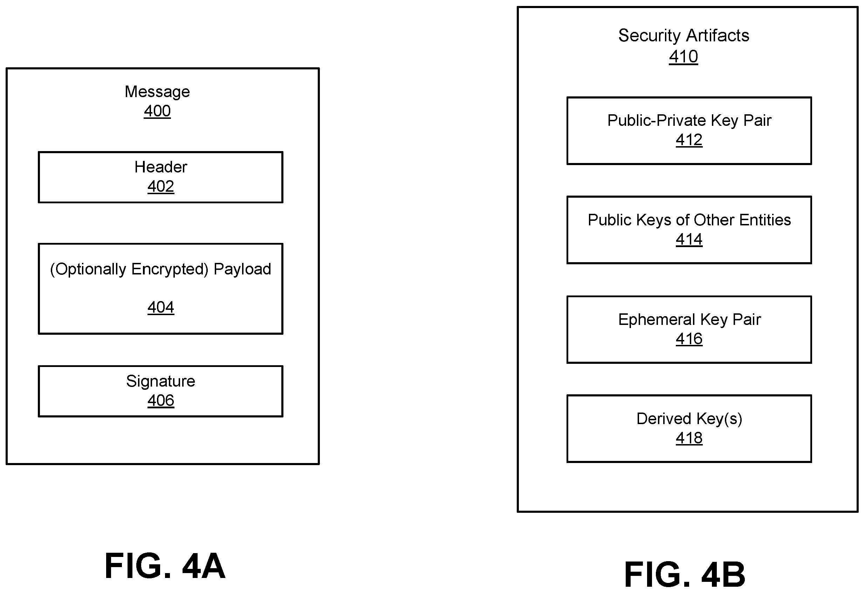

**FIG. 4A** is a high-level conceptual illustration of a message 400 and its contents, according to certain embodiments. Message 400 includes a header 402, a payload 404, and a digital signature 406. FIG. 4A is merely an example of information that may be contained in a message. As discussed below, messages can have different formats, so information present in one type of message may not be present in another type of message. For example, not all messages may include a signature or a payload.

Header 402 may include certain items of information needed by a receiver to process (e.g., decode) the message 400. For example, the header 402 may include metadata identifying the sender and the recipient, an indication of the length of the payload 404, and/or other information that is expected for the message 400 to comply with a particular communication protocol.

Payload 404 is the underlying message, which may include a text string or a block of data to be communicated to the recipient. As noted above, a stateless communication protocol may permit messages to be sent unencrypted, at least in some instances. Accordingly, encryption of the payload 404 may be optional. When the payload 404 is encrypted, the encryption key and the decryption key should be obtainable without using state information. One way to generate such keys is through ephemeral values, discussed below.

Signature 406 may include a digital value usable for verifying the integrity and authenticity of the message 400. For example, the signature 406 may include a hash value computed using SHA256 or some other hash algorithm. The input to the hash algorithm may include the entire message 400. In the case where the message 400 is to be sent encrypted, the signature 406 may be computed after encrypting the payload 404. The signature 406 may be encrypted using a private key of the sender (e.g., endpoint 210 in FIG. 3) and decrypted using the sender's corresponding public key. After decrypting the signature, the recipient can compute the hash using the same hash algorithm and compare the newly computed hash to the hash value from the decrypted signature. If the hash values match, this indicates that the message 400 has not been tampered with (e.g., modified after sending) and originated from the sender. In most instances, the recipient will have already obtained a copy of the sender's public key prior to receiving the message 400. For example, the public key of an electronic device may be included in the device certificate issued to the electronic device. Accordingly, the sender and the recipient may communicate their public keys by exchanging certificates (e.g., during a setup phase of establishing a communication channel between each other).

**FIG. 4B** illustrates an example of security artifacts 410 that may be used by a communication endpoint, according to certain embodiments. Security artifacts 410 may include a public-private key pair 412 belonging to the endpoint (e.g., an electronic device) and public keys 414 of other entities in the system. The public-private key pair 412 includes a public key and a corresponding private key. The public key portion of the public-private key pair 412 may be included in a certificate issued to the endpoint and shared with other entities (e.g., through exchanging of device certificates). As discussed above, the private key can be used to digitally sign messages when the endpoint is the sender. In some instances, a private key may also be used to sign and endorse messages sent by other entities. The public-private key pair 412 may be updated each time the endpoint is issued a new certificate. For example, electronic device 102 may generate a new public-private key pair 412 when its device certificate has expired or is about to expire. The electronic device 102 may then generate a certificate including the public key portion of the new public-private key pair and send the certificate to the CA 120 for signing.

Security artifacts 410 may further include an ephemeral key pair 416 and one or more derived keys 418. The ephemeral key pair 416 may include an ephemeral public key (also referred to herein as an ephemeral value) paired with an ephemeral private key. However, unlike the public-private key pair 412, ephemeral keys may be generated dynamically pursuant to a key exchange agreement between communication endpoints (e.g., using an Elliptic Curve Diffie-Hellman with Ephemeral keys (ECDHE) key exchange algorithm). In a stateless communication protocol, ephemeral keys may be message specific. When sending an encrypted message, the sender can generate a new ephemeral key pair for that message and use the private portion of the ephemeral key pair to derive an encryption key for encrypting the message payload. The public portion of the ephemeral key pair can be added to the message to enable the recipient to derive a corresponding decryption key. In some embodiments, the communication protocol may permit ephemeral key pairs to be reused under certain circumstances (e.g., when multiple encrypted messages need to be sent or received by a power-constrained device). Reuse of ephemeral key pairs avoids having to derive new keys for each message, resulting in power savings and less computational overhead.

Derived keys 418 may include encryption keys and decryption keys that are derived from ephemeral keys. In some embodiments, the derived keys 418 are generated using a key derivation process that produces a symmetric key usable as both an encryption key and a decryption key. On the sender side, the input to the key derivation process may include the ephemeral private key from the sender's ephemeral key pair 416 and the public key from the recipient's public-private key pair 412. On the receiver side, the input to the key derivation process may include the ephemeral public key from the sender's ephemeral key pair 416 and the private key from the recipient's public-private key pair 412. In either case, the same symmetric key is produced.

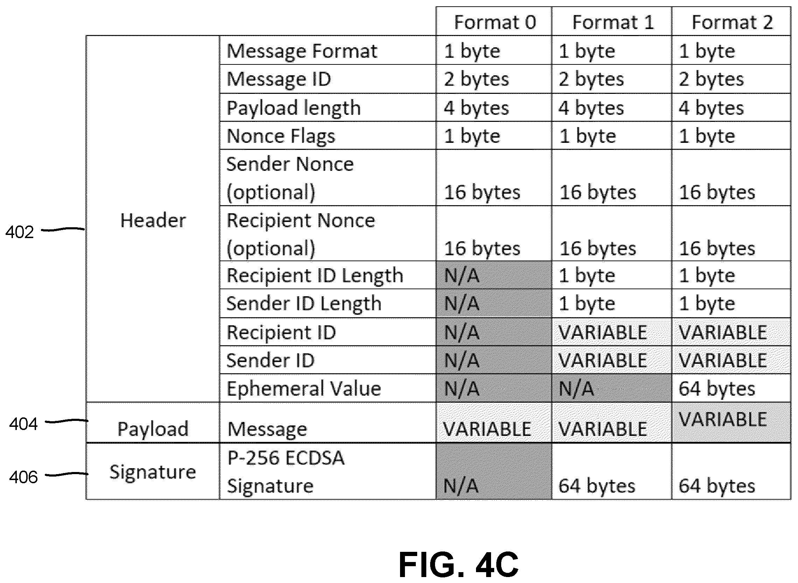

**FIG. 4C** illustrates an example of different message formats that may be available when communicating using a stateless and scalable communication protocol, according to certain embodiments. In the example of FIG. 4C, the communication protocol supports three message formats (Format 0, Format 1, and Format 2), each associated with a corresponding security level. However, the number of supported message formats may differ depending on implementation. As shown in FIG. 4C, the message formats have different requirements with respect to the header 402, the payload 404, and the signature 406 portion of a message. Each format supported by the communication protocol may be configured to carry a set of information that allows a message to be communicated at the corresponding security level and to be processed independently. For example, a Format 2 message includes an ephemeral value and possibly additional information needed by the recipient to decrypt the message. Any information that a recipient may require to successfully process a message can be included in the message itself and/or communicated during a setup phase when a communication channel is established. In this way, the recipient need not rely on information from other messages or maintain state information from one message to the next. Thus, a message can be processed even if an earlier message failed to be received.

Features that are common to all supported message formats may include certain fixed-sized fields in the header 402. For example, the header of each message 400 may begin with a 1-byte value indicating the format of the message (e.g., a value of 0, 1, or 2). A single byte can have 256 distinct values and therefore allows flexibility for additional message formats to be supported. The message format indicator can inform the recipient about how to interpret the message (e.g., the expected locations of various data elements).

Other header fields that are common to all message formats may include a message ID, a payload length indicator, and nonce flag bits. The message ID can be a numeric value identifying the message. A unique message ID may be assigned to each type of message that can possibly be transmitted. The payload length indicator specifies the length of the payload (e.g., in bytes). Some messages may have no payload (e.g., a payload length of 0 when the meaning of the message is conveyed by the message ID alone).

Nonce flag bits indicate whether sender and/or recipient nonces (sent by the sender or expected by the recipient) are present in the header. The nonce flag bits may, for example, correspond to the lowest (e.g., least significant) two bits of a 1-byte value. Nonces are one-time values that can be used for cryptography (e.g., to introduce entropy in a key derivation process). However, in some embodiments, nonces may optionally be included in the header 402 as a mechanism to prevent replay attacks rather than for cryptographic purposes.

In the illustrated example, Format 0 is the least secure message format and may be suitable for messages that do not require encryption or digital signature. Format 1 and Format 2 include fields for indicating the length (e.g., size in bytes) of a recipient ID and a sender ID. Sender and recipient IDs are mandatory for Format 1 and Format 2 and may be variable-length values identifying the sender and the recipient of a message. In contrast, a Format 0 message has no sender ID or recipient ID. As indicated in FIG. 4C, a Format 0 message also has no ephemeral public key or signature.

Format 1 includes a signature field and therefore provides a greater level of security than Format 0. Thus, Format 1 may be used for messages that need to be signed but not encrypted. In some embodiments, the signature field may be calculated using an Elliptic Curve Digital Signature Algorithm (ECDSA) in combination with Secure Hash Algorithm 256 (SHA-256) hashing.

Format 2 includes the same signature field as Format 1 but adds an ephemeral value (public key). As discussed above, an ephemeral public key can be used on the receiver side to derive a symmetric key for decrypting a message. Thus, Format 2 may be used for messages that need to be encrypted as well as signed.

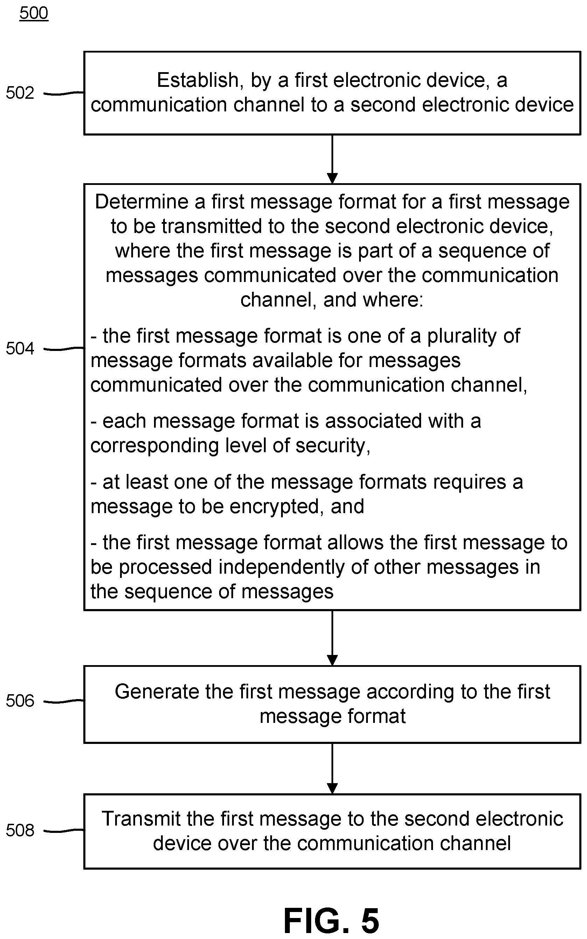

**FIG. 5** is a flow diagram of a method 500 for communicating over a communication channel, according to certain embodiments. The method 500 may be performed by a first electronic device (e.g., the electronic device 102) that is preparing to send one or more messages to a second electronic device (e.g., the electronic device 104 or some other communication endpoint in the system of FIG. 1).

At block 502, the first electronic device may establish a communication channel to the second electronic device. The communication channel can be a wired or wireless channel in which messages are transmitted and received in accordance with a stateless and scalable communication protocol. In some embodiments, the communication channel may be established after mutual authentication between the first electronic device and the second electronic device. The authentication mechanism may be provided by a PKI in which the first electronic device and the second electronic device are enrolled.

At block 504, the first electronic device may determine a first message format for a first message to be transmitted to the second electronic device. The first message may be part of a sequence of messages communicated over the communication channel. In the context of FIG. 5, the first message is not necessarily the beginning of the sequence of messages. Rather, the first message may correspond to any message transmitted by the first electronic device over a period in which two or more messages are communicated between the first electronic device and the second electronic device. The sequence of messages may include messages transmitted by the first electronic message, messages transmitted by the second electronic device, or both.

As discussed above, a scalable communication protocol may permit messages to be communicated with varying levels of security. Thus, the first message format may be one of a plurality of message formats available for messages communicated over the communication channel, with at least one message format in the plurality of message formats requiring a message to be encrypted.

The message format to use for a particular message may be predetermined. For example, the communication protocol may specify a corresponding format for each type of message that can possibly be transmitted within the system. Thus, each message ID may be associated with a particular message format. For example, a first group of message IDs may be assigned to Format 0, a second group of message IDs assigned to Format 1, and a third group of message IDs assigned to Format 2. In some embodiments, the first electronic device may refer to a list that enumerates possible message types (e.g., a list arranged according to message ID). The list may be stored locally as a table or other data structure and may specify a corresponding message format for each message type.

In some embodiments, the communication protocol may permit the first electronic device to choose which format to use for a particular message. For instance, the first electronic device can determine a security level to be applied to the first message based on any number of factors including, for example, the sensitivity of the data in the first message, a status of the first electronic device and/or a status of the second electronic device (e.g., whether one or more of the devices is operating in a power-saving mode), and the type of message being communicated. One or more of the available message formats may be configured to allow the first message to be communicated at the determined security level. In that case, the first electronic device may select the first message format from among the message formats that support the determined security level. Examples of different security levels include: (1) no encryption and no digital signature, (2) encryption using a first encryption algorithm and no digital signature, (3) encryption using the first encryption algorithm with an included signature, (4) encryption using a second encryption algorithm different from the first encryption algorithm and with an included signature, and so on. Other security configurations are possible.

To support a particular security level, a message format may include one or more fields dedicated to holding information that a recipient needs in order to process a message transmitted at that particular security level and independently of other messages. For example, if the first electronic device and the second electronic device have yet to agree on which cryptographic algorithm to use and the first message is to be encrypted, the first message format may include a field for identifying a cryptographic algorithm. In some instances, the first message format may include one or more security artifacts, examples of which include ephemeral values and digital signatures, as discussed above. In other instances, the first message format may include other types of security artifacts or may not include any security artifacts whatsoever (e.g., when the first message is unsigned and unencrypted).

At block 506, the first electronic device may generate the first message according to the first message format. As discussed above, each message format supported by the communication protocol may be configured to allow a message to be processed independently, irrespective of an order in which the message is received relative to an order in which the message is transmitted. For example, the first message format may, by virtue of carrying information needed by the second electronic device to process the first message (e.g., any necessary information that hasn't already been communicated), allow the first message to be processed independently of other messages in the sequence of messages communicated over the communication channel. Depending on the first message format determined in block 504, the first message may be generated as a digitally signed message or an unsigned message. Further, the first message may be generated as an unencrypted message or an encrypted message (e.g., a message that is both signed and encrypted, or a message that is encrypted but unsigned).

Generating the first message may involve configuring a header of the first message to conform to the first message format. For example, the header may be configured to include an indication that the first message has the first message format. In some embodiments, the format indicator may be located at the beginning of the header (e.g., as shown in FIG. 4C). Other parts of the first message may also be configured according to the first message format. For example, if the first message is to be encrypted, the first electronic device may generate an ephemeral key pair including an ephemeral public key and an ephemeral private key, encrypt a payload section of the first message (e.g., using a derived symmetric key as discussed above), and add the ephemeral public key to the first message.

At block 508, the first electronic device may transmit the first message to the second electronic device over the communication channel.

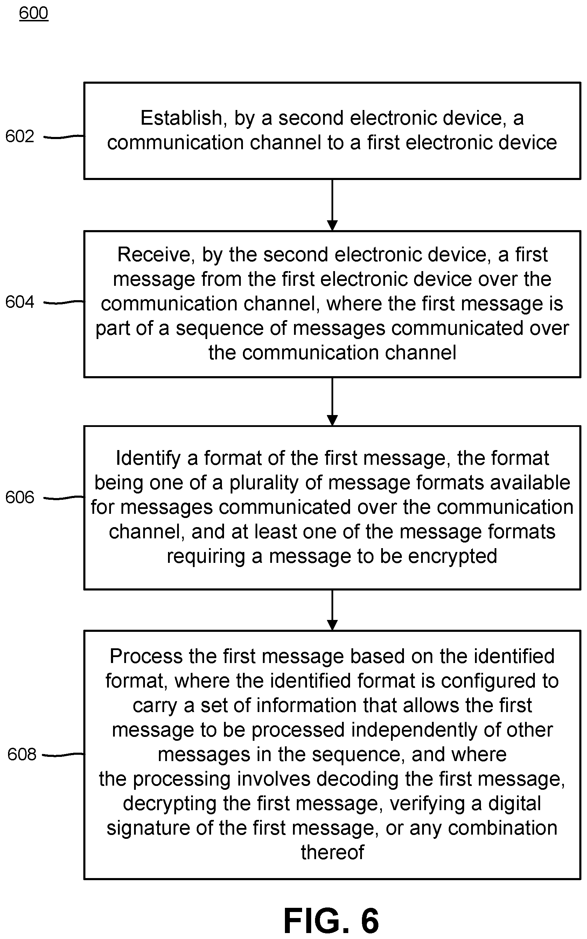

**FIG. 6** is a flow diagram of a method 600 for communicating over a communication channel, according to certain embodiments. The method 600 may be performed by a second electronic device (e.g., the electronic device 104) that is expecting to receive one or more messages from a first electronic device (e.g., the electronic device 102 or some other communication endpoint in the system of FIG. 1). In particular, the method 600 may correspond to steps performed by the second electronic device in the method 500 of FIG. 5. As indicated in the discussion below, the method 600 may include functionality similar or analogous to functionality described with respect to the first electronic device in FIG. 5. For the sake of brevity, details of such functionality are not repeated. For example, block 602 of FIG. 6 may correspond to block 502.

At block 602, the second electronic device may establish a communication channel to the first electronic device. The communication channel can be a wired or wireless channel in which messages are transmitted and received in accordance with a stateless and scalable communication protocol.

At block 604, the second electronic device may receive a first message from the first electronic device over the communication channel. The first message is part of a sequence of messages communicated over the communication channel and may, for example, correspond to the message transmitted in block 508 of FIG. 5.

At block 606, the second electronic device may identify a format of the first message. The format of the first message may be one of a plurality of message formats available for messages communicated over the communication channel. For example, the format of the first message may be one of several message formats supported by the communication protocol, with each supported format being configured to allow a message to be processed independently. Further, each message format may be associated with a corresponding level of security, and at least one of the message formats may require that a message be encrypted.

At block 608, the second electronic device may process the first message based on the format identified in block 606. Depending on the message format, the processing in block 608 may involve decoding the first message, decrypting the first message, verifying a digital signature of the first message, or any combination thereof. Using FIG. 4C as an example, a Format 0 message would be decoded but not decrypted or subjected to signature verification. A Format 1 message would be decoded and signature verified. A Format 2 message would be decoded, signature verified, and decrypted. Other operations may be performed as part of processing the first message (e.g., extracting a payload, recipient ID, or sender ID).

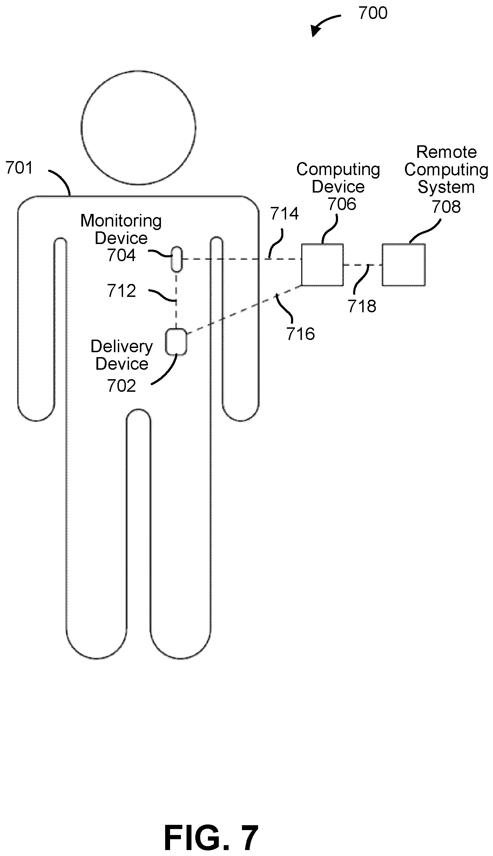

**FIG. 7** illustrates an example of a therapy delivery system 700 for a person 701. The system 700 may correspond to an embodiment of the system 100 in FIG. 1. In some embodiments, the system 700 may be an insulin delivery system, and the person 701 may be a diabetes patient. The system 700 may include a delivery device 702, a monitoring device 704, a computing device 706, and a remote or cloud computing system 708. The delivery device 702, the monitoring device 704, and the computing device 706 may be embodied in various ways, including being disposed in one or more device housings. For example, in some embodiments, all the devices 702-706 may be disposed in a single device housing. In some embodiments, each of the devices 702-706 may be disposed in a separate device housing. In some embodiments, two or more of the devices 702-706 may be disposed in the same device housing, and/or a single device 702, 704, or 706 may have two or more parts that are disposed in two or more housings. For example, the monitoring device 704 may include an on-body part and a display and control part communicating with the on-body part through wires or wirelessly. Similarly, the delivery device 702 may include an on-body site (e.g., including a cannula) and a part that includes a reservoir, a pump, and a control unit. These and other embodiments, and combinations thereof, are contemplated to be within the scope of the present disclosure.

System 700 may include a plurality of communications links, such as communication links 712-718. The communications links 712-718 may each be a wired connection and/or a wireless connection. In embodiments where two devices are located in the same device housing, the communication link may include, for example, wires, cables, and/or communication buses on a printed circuit board, among other things. In embodiments where two devices are separated from each other in different device housings, the communication links may be wired and/or wireless connections. Wired connections may include, without limitation, an Ethernet connection, a Universal Serial Bus (USB) connection, and/or another type of physical connection. Wireless connections may include, without limitation, a cellular connection, a Wi-Fi connection, a Bluetooth^{®} connection, a mesh network connection, and/or another type of connection using a wireless communication protocol. Some embodiments of the communication links 712-718 may use direct connections, such as Bluetooth^{®} connections, and/or may use connections that route through one or more networks or network devices (not shown), such as an Ethernet network, a Wi-Fi network, a cellular network, a satellite network, an intranet, an extranet, the Internet, and/or the Internet backbone, among other types of networks. Various combinations of wired and/or wireless connections may be used for the communication links 712-718.

Delivery device 702 is configured to deliver a therapeutic substance (e.g., insulin) to the person 701. The delivery device 702 may be secured to the person 701 (e.g., to the body or clothing of the person 701) or may be implanted on or in the body of the person 701. In some embodiments, the delivery device 702 may include a reservoir, an actuator, a delivery mechanism, and a cannula (not shown). The reservoir may be configured to store an amount of the therapeutic substance. In some embodiments, the reservoir may be refillable or replaceable. The actuator may be configured to drive the delivery mechanism. In some examples, the actuator may include a motor, such as an electric motor. The delivery mechanism may be configured to move the therapeutic substance from the reservoir through the cannula. In some examples, the delivery mechanism may include a pump and/or a plunger. The cannula may facilitate a fluidic connection between the reservoir and the body of the person 701. The cannula and/or a needle may facilitate delivery of the therapeutic substance to a tissue layer, vein, or body cavity of the person 701. During operation, the actuator, in response to a signal (e.g., a command signal), may drive the delivery mechanism, thereby causing the therapeutic substance to move from the reservoir, through the cannula, and into the body of the person 701.

The components of the delivery device 702 described above are merely provided as an example. The delivery device 702 may include other components, such as, without limitation, a power supply, a communication transceiver, one or more processors or other computing resources, memory devices, and/or user interfaces, among other things. Persons skilled in the art will recognize various implementations of the delivery device 702 and the components of such implementations. All such implementations and components are contemplated to be within the scope of the present disclosure.

Monitoring device 704 is configured to detect a physiological condition (e.g., a glucose concentration level) of the person 701 and may also be configured to detect other things. The monitoring device 704 may be secured to the body of the person 701 (e.g., to the skin of person 701 via an adhesive) and/or may be at least partially implanted into the body of the person 701. Depending on the particular location or configuration, the monitoring device 704 may be in contact with biological matter (e.g., interstitial fluid and/or blood) of the person 701. The monitoring device 704 may include one or more sensors (not shown), such as electrochemical sensors, electrical sensors, and/or optical sensors.

As persons skilled in the art will understand, an electrochemical sensor may be configured to respond to the interaction or binding of a biological marker to electrodes by generating an electrical signal based on, for example, a potential, conductance, current, and/or impedance of an electrical path through the electrodes. The electrodes may include a material selected to interact with a particular biomarker, such as glucose. The potential, current, conductance, and/or impedance may correlate with a concentration of the particular biomarker. In one example, the electrochemical sensor may include a glucose limiting membrane (GLM) that limits the amount of glucose and oxygen delivered to a glucose oxidase (GOx) layer of a working electrode of the sensor to ensure that the reactions are glucose limited. The GOx layer or another active enzyme layer on the working electrode of the sensor may break down glucose and oxygen into gluconic acid and hydrogen peroxide. The generated peroxide molecules may interact with the working electrode to break down hydrogen peroxide into two hydrogen ions, oxygen, and two electrons at the surface of the working electrode. When a voltage signal is supplied, the electrical charges may be forced to move, thereby generating a sensor current signal (Isig) that can be measured by sensor electronics. Other signals such as a counter electrode voltage (Vcntr), electrochemical impedance spectroscopy (EIS) at different frequencies, and the like, may also be measured. The signals measured using the sensor, including the Isig, Vcntr, and EIS, may be processed (e.g., filtered or transformed) to generate some other signals or parameters, such as filtered Isig signals, real and imaginary impedance at various frequencies, and the like. These signals and/or the processed parameters may be used in one or more sensor glucose (SG) models (e.g., machine learning models or mathematical models) to determine SG values that may be estimations of the blood glucose (BG) levels of the patient.

As persons skilled in the art would understand, an electrical sensor may be configured to respond to an electrical biosignal by generating an electrical signal based on an amplitude, frequency, and/or phase of the electrical biosignal. The electrical biosignal may include a change in electric current produced by the sum of an electrical potential difference across a tissue, such as the nervous system, of the person 701. In some embodiments, the electrical biosignal may include portions of a potential change produced by the heart of the person 701 over time (e.g., recorded as an electrocardiogram) that may be indicative of a glucose level of the person 701. An optical sensor may be configured to, for example, respond to the interaction or binding of a biological marker to a substrate by generating an electrical signal based on change in luminance of the substrate. In one example, the substrate may include a material selected to fluoresce in response to contact with a selected biomarker, such as glucose. The fluorescence may be proportional to a concentration of the selected biomarker.

In some embodiments, the monitoring device 704 may include other types of sensors that may be worn, carried, or coupled to the person 701 to measure activity of the person 701 that may influence the glucose levels or glycemic response of the person 701. As an example, the sensors may include an acceleration sensor configured to detect an acceleration of the person 701 or a portion of the person 701, such as the person's hands or feet, the position changes of which may be associated with an activity of person 701. For example, the acceleration or movement (or lack thereof) of the body portion of person 701 may be indicative of exercise, sleep, or food/beverage consumption activity of person 701, which may influence the glycemic response of person 701. In some embodiments, the sensors may measure heart rate and/or body temperature, which may indicate an amount of physical exertion experienced by person 701. In some embodiments, the sensors may include a Global Positioning System (GPS) receiver which may detect GPS signals to determine a location of person 701.

The sensors described above are merely provided as examples. Other sensors or types of sensors for monitoring physiological condition, activity, and/or location, among other things, will be recognized by persons skilled in the art and are contemplated to be within the scope of the present disclosure. For any sensor, a signal provided by the sensor shall be referred to as a "sensor signal." The term "sensed data" may mean and include the information represented by a sensor signal or by a pre-processed sensor signal. In some embodiments, sensed data may include glucose levels of person 701, acceleration of a part of person 701, heart rate of person 701, temperature of person 701, and/or geolocation (e.g., GPS location) of person 701, among other things. Monitoring device 704 may communicate sensed data to delivery device 702 via communication link 712 and/or to computing device 706 via communication link 714. Use of sensed data by delivery device 702 and/or by computing device 706 is described in more detail below.

The monitoring device 704 may include components and/or circuitry configured to pre-process sensor signals. Pre-processing may include, for example, amplification, filtering, attenuation, scaling, isolation, normalization, transformation, sampling, and/or analog-to-digital conversion, among other things. Persons skilled in the art will recognize various implementations for such pre-processing, including, without limitation, implementations using processors, controllers, integrated circuits, application specific integrated circuits (ASICs), hardware, firmware, programmable logic devices, and/or machine-executable instructions, among others. The types of pre-processing and their implementations are merely provided as examples. Other types of pre-processing and implementations are contemplated to be within the scope of the present disclosure. In some embodiments, the monitoring device 704 may not perform pre-processing.

Computing device 706 provides processing capabilities and may be implemented in various ways. In some embodiments, the computing device 706 may be a consumer device, such as a smartphone, a computerized wearable device (e.g., a smartwatch), a tablet computer, a laptop computer, or a desktop computer, among others, or may be a special purpose device (e.g., a portable control device) provided by, for example, the manufacturer of the delivery device 702. In some embodiments, the computing device 706 may be processing circuitry that is integrated with another device, such as the delivery device 702. In some embodiments, the computing device 706 may be secured to the person 701 (e.g., to the body or clothing of person 701), may be at least partially implanted into the body of person 701, and/or may be held by the person 701.

For each of the embodiments of the computing device 706, the computing device 706 may include various types of logic circuitry, including, but not limited to, microprocessors, controllers, digital signal processors (DSPs), application specific integrated circuits (ASICs), field-programmable gate arrays (FPGAs), central processing units (CPU), graphics processing units (GPU), programmable logic devices, memory (e.g., random access memory, volatile memory, non-volatile memory, etc.), or other discrete or integrated logic circuitry, as well as combinations of such components. The term "processing circuitry" may generally refer to any of the foregoing logic circuitry, alone or in combination with other logic circuitry, or any other circuitry for performing computations.

One or more of the devices 702-706 may include a user interface (not shown) that presents information to the person 701 and/or receives information from the person 701. The user interface may include a graphical user interface (GUI), a display device, a keyboard, a touchscreen, a speaker, a microphone, a vibration motor, buttons, switches, and/or other types of user interfaces. Persons skilled in the art will recognize various types of user interfaces that may be used, and all such user interfaces are contemplated to be within the scope of the present disclosure. For example, where the computing device 706 is a consumer device such as a smart phone, tablet computer, laptop computer, or the like, the user interfaces would include a display device, a physical and/or virtual keyboard, and/or audio speakers provided by such consumer devices, among other things. In some embodiments, a user interface may notify the person 701 of sensed data (e.g., glucose level) and/or insulin delivery data (e.g., rates of historic, current, or future insulin delivery) and may present alerts to the person 701. In some embodiments, a user interface may receive inputs from the person 701, which may include, for example, a requested change in insulin delivery and/or a meal indication, among other things. The descriptions and embodiments above regarding user interfaces are merely provided as examples, and other types and other uses of user interfaces are contemplated to be within the scope of the present disclosure.

In one specific example, communications between the devices 702-706 and cooperation between the devices 702-706 may be used for insulin delivery. As discussed above, the devices 702-706 may communicate with each other via communication links 712-716. In some embodiments, the computing device 706 may control operation of the delivery device 702 and/or the monitoring device 704. For example, the computing device 706 may generate one or more signals (e.g., a command signal) that cause the delivery device 702 to deliver insulin to the person 701, for example, as a basal dosage and/or a bolus dosage. In some embodiments, the computing device 706 may receive data associated with insulin delivery (e.g., insulin delivery data) from the delivery device 702 and/or receive sensed data (e.g., glucose levels) from the monitoring device 704 and may perform computations based on the insulin delivery data, the sensed data, and/or other data to control the delivery device 702. Insulin delivery data may include, but is not limited to, a type of insulin being delivered, historical insulin delivery rates and/or amounts, current insulin delivery rate and/or amount, and/or user input affecting insulin delivery. As persons skilled in the art will understand, in a closed-loop operating mode, computing device 1406 may communicate dosage commands to the delivery device 702 based on a difference between a current glucose level in the body of the person 701 (e.g., received from the monitoring device 704) and a target glucose level (e.g., determined by the computing device 706). The dosage commands may indicate an amount of insulin to be delivered and/or a rate of insulin delivery and may regulate the current glucose level toward the target glucose level.

Remote or cloud computing system 708 may be a proprietary remote/cloud computing system or a commercial cloud computing system including one or more server computing devices. The remote/cloud computing system 708 may provide additional computing resources on-demand as needed when the computing resources of a client computing device (e.g., the computing device 706) are not sufficient. The computing device 706 and the remote/cloud computing system 708 may communicate with each other through a communication link 718, which may traverse one or more communication networks (not shown). The communication networks may include, without limitation, an Ethernet network, Wi-Fi network, a cellular network, a satellite network, an intranet, an extranet, the Internet, and/or the Internet backbone, among other types of networks. Persons skilled in the art will recognize implementations for the remote/cloud computing system 708 and how to interface with such systems through various types of networks. For example, the remote/cloud computing system 708 may include an array of processing circuitry and may execute machine-readable instructions. Such implementations, interfaces, and networks are contemplated to be within the scope of the present disclosure.

In some embodiments, therapy may be effected based on communicating a therapy determination toward a therapy delivery device. Using FIG. 7 as an example, a therapy determination (e.g., an insulin amount) may be made by the remote/cloud computing system 708 and communicated to the delivery device 702 via the computing device 706. Alternatively, the therapy determination may be made by the monitoring device 704 and communicated to the delivery device 702 directly or through an intermediary such as the computing device 706. In some cases, the computing device 706 may be the source of the therapy determination.

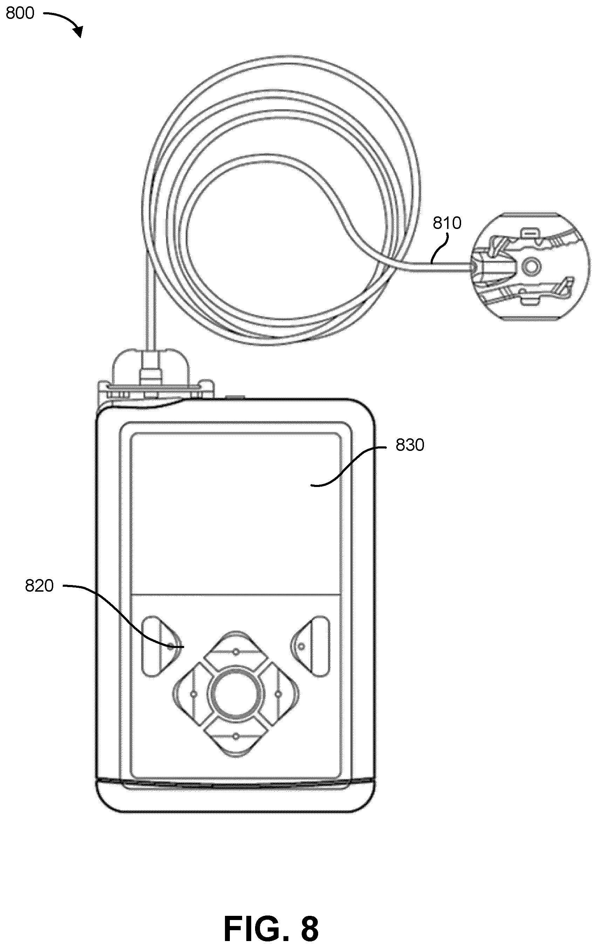

**FIG. 8** illustrates an example of a delivery device 800, which may implement a delivery device described herein (e.g., the delivery device 702). Delivery of a therapeutic agent may be performed based on internal communication between a central computing module (e.g., a microprocessor of the delivery device 800) and a delivery module (e.g., including a microcontroller, a motor, and a pump). For instance, insulin delivery may be caused by the central computing module communicating a delivery command in the form of an electrical signal that travels via a communication fabric to the delivery module. The central computing module may also be configured to communicate (e.g., via a transceiver) with a computing device (e.g., the computing device 706). In turn, the computing device may be communicatively coupled to a remote or cloud computing system (e.g., remote/cloud computing system 708). The delivery device 800 may communicate various event data toward the remote or cloud computing system, which may communicate delivery determinations toward the delivery device 800, in accordance with the techniques described herein.

The delivery device 800 may provide insulin (or some other therapeutic agent) through a small tube 810 configured for fluidic connection with a cannula inserted subcutaneously. In embodiments where delivery device 800 is an insulin delivery device, the delivery device 800 may be configured to deliver two types of dosages-a basal dosage, which can be delivered periodically (e.g., every five minutes) in tiny amounts throughout the day and night, and a bolus dosage to cover an increase in blood glucose from meals and/or to correct high blood glucose levels. Delivery device 800 may include a user interface having button elements 820 that can be manipulated to administer a dose, to change therapy settings, to change user preferences, to select display features, and the like. The delivery device 800 may also include a display device 830 that can be used to present various types of information or data to the user. In accordance with aspects of the present disclosure, a user of the delivery device 800 may use the button elements 820 to input certain event data (e.g., event type, event start time, event details, etc.), and the user inputs can be confirmed using the display device 830.

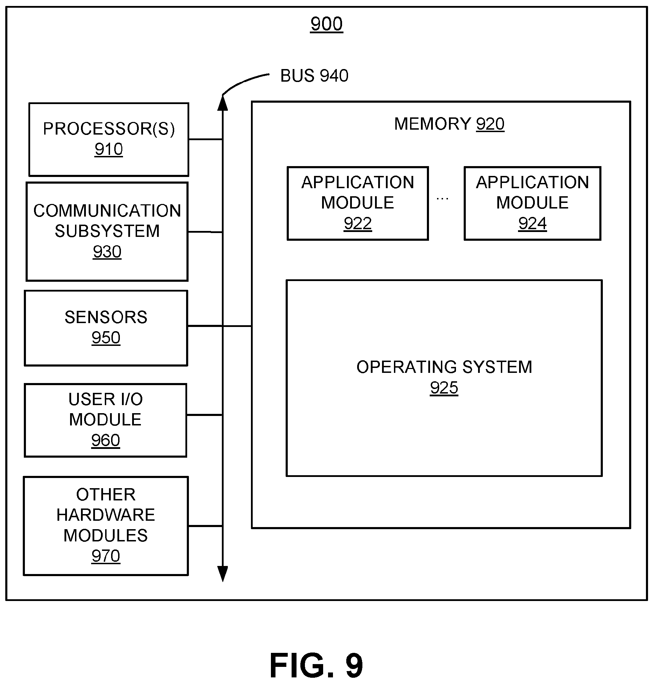

**FIG. 9** is a simplified block diagram of an example electronic device 900 that may implement some of the examples disclosed herein. For example, electronic device 900 may correspond to electronic device 102, electronic device 104, remote computing system 130, or remote computing system 140 in FIG. 1). In the illustrated example, electronic device 900 may include one or more processor(s) 910 and memory 920. Processor(s) 910 may be configured to execute instructions stored in memory 920 for performing one or more of the methods described herein and other applications. Processor(s) 910 may include, for example, one or more central processing units, microprocessors, microcontrollers, special-purpose processors (e.g., digital signal processors), ASICs, DSPs, FPGAs, or other processors suitable for implementation within a portable electronic device. Processor(s) 910 may be communicatively coupled with a plurality of components within electronic device 900. To realize this communicative coupling, processor(s) 910 may communicate with the other illustrated components across a bus 940. Bus 940 may be any subsystem adapted to transfer data within electronic device 900. Bus 940 may include a plurality of computer buses and additional circuitry to transfer data.

Memory 920 may include one or more transitory and/or non-transitory storage devices, such as, for example, a static random access memory (SRAM), a dynamic random access memory (DRAM), a read-access memory (RAM), a read-only memory (ROM), a programmable read-only memory (PROM), an erasable programmable read-only memory (EPROM), a FLASH-EPROM, a secure digital (SD) card, and any other memory chip or cartridge. Such storage devices may be configured to implement any appropriate data stores, including without limitation, various file systems, data structures, computer-readable instructions, program modules, and the like. In some embodiments, memory 920 may be distributed into different hardware modules.

Memory 920 may include an operating system 925 loaded therein. Operating system 925 may be operable to initiate the execution of the instructions provided by application modules 922-924 and/or manage other hardware modules 970, as well as interface with a communication subsystem 930 which may include one or more wired and/or wireless transceivers. Operating system 925 may be adapted to perform other operations across the components of electronic device 900 including threading, virtualization, resource management, data storage control, and other similar functionality. In some embodiments, memory 920 may store a plurality of application modules 922 through 924, which may include any number of applications. Examples of the applications may include an insulin calculator, a blood glucose level monitor or predictor, a glucose level management application, and the like. Application modules 922-924 may include particular instructions to be executed by processor(s) 910. In some embodiments, certain applications or parts of application modules 922-924 may be executable by other hardware modules 970.

Communication subsystem 930 may include, for example, an infrared communication device, a wireless communication device and/or chipset (such as a Bluetooth^{®} device, an IEEE 802.11 device, a Wi-Fi device, a WiMax device, cellular communication devices, etc.), and/or similar communication interfaces. One or more antennas (not shown) may be used for wireless communication as part of communication subsystem 930 or as a separate component coupled to any portion of electronic device 900, such as a wireless charging receiver or a near-field communication receiver. In some embodiments, communication subsystem 930 may include circuits for wired communication technologies, such as Ethernet, coaxial communications, universal serial bus (USB), and the like. Communications subsystem 930 may permit data to be exchanged with a network, other computer systems, and/or any other devices. For example, communications subsystem 930 may be used to receive therapy determinations for therapeutic fluid (e.g., insulin) delivery, such as from a cloud computing system via an intermediary computing device (e.g., a controller) communicatively coupled to electronic device 900, where processor(s) 910 may, based on the therapy determinations, send commands to an actuator controller to cause the delivery of appropriate amounts of therapeutic fluid (e.g., insulin) to a user. In another example, communications subsystem 930 may be used to communicate measurement results (e.g., sensor glucose levels) to a computing device (e.g., a smartphone or a personal health monitoring device) and/or to a remote server via the computing device, or receive data (e.g., calibration data, configuration data, etc.) from the computing device or the remote server via the computing device.

Sensor(s) 950 may include, for example, a camera, an infrared sensor, an accelerometer, a pressure sensor, a temperature sensor, a proximity sensor, a magnetometer, a gyroscope, an inertial sensor (e.g., an inertial measurement unit (IMU)), an ambient light sensor, a position sensor, a depth sensor, a gesture detector, or any other similar module operable to provide sensory output and/or receive sensory input. In one example, one or more sensors of the electronic device 900 may be configured to measure a physiological parameter. For example, sensor(s) 950 may include an interstitial or blood glucose sensor.

Input/output user interface 960 may allow a user to send action requests to electronic device 900 to perform particular actions, and may provide information (e.g., status of electronic device 900, measurement results, alerts, etc.) to the user. Input/output user interface 960 may include one or more input devices, such as, for example, a touchscreen, a touch pad, microphone(s), button(s), dial(s), switch(es), a keyboard, a mouse, a game controller, or any other suitable device for receiving action requests and communicating the received action requests to processor(s) 910. In some embodiments, input/output user interface 960 may include one or more output devices, such as a display, a speaker, a light emitting device, a haptic device, and the like, to provide feedback to or alert the user.

In some embodiments, electronic device 900 may include a plurality of other hardware modules 970. Each of other hardware modules 970 may be a physical module within electronic device 900. While each of other hardware modules 970 may be permanently configured as a structure, some of other hardware modules 970 may be temporarily configured to perform specific functions or temporarily activated. Examples of other hardware modules 970 may include, for example, an audio output and/or input module (e.g., a microphone or speaker), a near field communication (NFC) module, a rechargeable battery, a battery management system, a wired/wireless battery charging system, an actuator controller, and the like. In some embodiments, one or more functions of other hardware modules 970 may be implemented in software.

In one example, electronic device 900 may be part of an insulin delivery device (e.g., a pump) that can deliver fast-acting insulin through a small tube configured for fluidic connection with a cannula inserted subcutaneously. Electronic device 900 may cause the delivery of two types of dosages-a basal dosage, which can be delivered periodically (e.g., every five minutes) in tiny amounts throughout the day and night, and a bolus dosage to cover an increase in blood glucose from meals and/or to correct high blood glucose levels. The insulin delivery device may include a user interface having button elements that can be manipulated to administer a bolus of insulin, to change therapy settings, to change user preferences, to select display features, and the like. The insulin delivery device may also include a display device that can be used to present various types of information or data to the user. In accordance with aspects of the present disclosure, a user of the insulin delivery device may use the button elements to input certain event data (e.g., event type, event start time, event details, etc.), and the user inputs can be confirmed using the display device.

In various implementations, the above-described hardware and modules may be implemented on a single device or on multiple devices that can communicate with one another using wired or wireless connections. In alternative configurations, different and/or additional components may be included in electronic device 900. Similarly, functionality of one or more of the components can be distributed among the components in a manner different from the manner described above.

The methods, systems, and devices discussed above are examples. Various embodiments may omit, substitute, or add various procedures or components as appropriate. For instance, in alternative configurations, the methods described may be performed in an order different from that described, and/or various stages may be added, omitted, and/or combined. Also, features described with respect to certain embodiments may be combined in various other embodiments. Different aspects and elements of the embodiments may be combined in a similar manner. Also, technology evolves and, thus, many of the elements are examples that do not limit the scope of the disclosure to those specific examples.

Specific details are given in the description to provide a thorough understanding of the embodiments. However, embodiments may be practiced without these specific details. For example, well-known circuits, processes, systems, structures, and techniques have been shown without unnecessary detail in order to avoid obscuring the embodiments. This description provides example embodiments only, and is not intended to limit the scope, applicability, or configuration of the invention. Rather, the preceding description of the embodiments will provide those skilled in the art with an enabling description for implementing various embodiments. Various changes may be made in the function and arrangement of elements without departing from the spirit and scope of the present disclosure.

Also, some embodiments were described as processes depicted as flow diagrams or block diagrams. Although each may describe the operations as a sequential process, many of the operations may be performed in parallel or concurrently. In addition, the order of the operations may be rearranged. A process may have additional steps not included in the figure. Furthermore, embodiments of the methods may be implemented by hardware, software, firmware, middleware, microcode, hardware description languages, or any combination thereof. When implemented in software, firmware, middleware, or microcode, the program code or code segments to perform the associated tasks may be stored in a computer-readable medium such as a storage medium. Processors may perform the associated tasks.

It will be apparent to those skilled in the art that substantial variations may be made in accordance with specific requirements. For example, customized or special-purpose hardware might also be used, and/or particular elements might be implemented in hardware, software (including portable software, such as applets, etc.), or both. Further, connection to other computing devices such as network input/output devices may be employed.

The embodiments disclosed herein are examples of the disclosure and may be embodied in various forms. For instance, although certain embodiments herein are described as separate embodiments, each of the embodiments herein may be combined with one or more of the other embodiments herein. Specific structural and functional details disclosed herein are not to be interpreted as limiting, but as a basis for the claims and as a representative basis for teaching one skilled in the art to variously employ the present disclosure in virtually any appropriately detailed structure. Like reference numerals may refer to like elements throughout the description of the figures.

Any of the herein described techniques, operations, methods, programs, algorithms, or codes may be converted to, or expressed in, a programming language or computer program embodied on a computer, processor, or machine-readable medium. The terms "programming language" and "computer program," as used herein, each include any language used to specify instructions to a computer or processor, and include (but is not limited to) the following languages and their derivatives: Assembler, Basic, Batch files, BCPL, C, C+, C++, Delphi, Fortran, Java, JavaScript, machine code, operating system command languages, Pascal, Perl, PL1, Python, scripting languages, Visual Basic, metalanguages which themselves specify programs, and all first, second, third, fourth, fifth, or further generation computer languages. Also included are database and other data schemas, and any other meta-languages. No distinction is made between languages which are interpreted, compiled, or use both compiled and interpreted approaches. No distinction is made between compiled and source versions of a program. Thus, reference to a program, where the programming language could exist in more than one state (such as source, compiled, object, or linked) is a reference to any and all such states. Reference to a program may encompass the actual instructions and/or the intent of those instructions.

It should be understood that the foregoing description is only illustrative of the present disclosure. To the extent consistent, any or all of the aspects detailed herein may be used in conjunction with any or all of the other aspects detailed herein. Various alternatives and modifications can be devised by those skilled in the art without departing from the disclosure. Accordingly, the present disclosure is intended to embrace all such alternatives, modifications, and variances. The embodiments described with reference to the attached drawing figures are presented only to demonstrate certain examples of the disclosure. Other elements, steps, methods, and techniques that are insubstantially different from those described above and/or in the appended claims are also intended to be within the scope of the disclosure. While several embodiments of the disclosure have been depicted in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto. The aspects and features of the present disclosure and may be embodied in various forms. Thus, specific structural and functional details disclosed herein are not to be interpreted as limiting, but as a basis for the claims and as a representative basis for teaching one skilled in the art to variously employ the present disclosure in virtually any appropriately detailed structure.

With reference to the appended figures, components that can include memory can include non-transitory machine-readable media. The term "machine-readable medium," "processor-readable medium," or "computer-readable medium" may refer to any storage medium that participates in providing data that causes a machine to operate in a specific fashion. In embodiments provided hereinabove, various machine-readable media might be involved in providing instructions/code to processing units and/or other device(s) for execution. Additionally or alternatively, the machine-readable media might be used to store and/or carry such instructions/code. In many implementations, a computer-readable medium is a physical and/or tangible storage medium. Such a medium may take many forms, including, but not limited to, non-volatile media, volatile media, and transmission media. Common forms of computer-readable media include, for example, magnetic and/or optical media such as compact disk (CD) or digital versatile disk (DVD), punch cards, paper tape, any other physical medium with patterns of holes, a RAM, a programmable read-only memory (PROM), an erasable programmable read-only memory (EPROM), a FLASH-EPROM, any other memory chip or cartridge, a carrier wave as described hereinafter, or any other medium from which a computer can read instructions and/or code. A computer program product may include code and/or machine-executable instructions that may represent a procedure, a function, a subprogram, a program, a routine, an application (App), a subroutine, a module, a software package, a class, or any combination of instructions, data structures, or program statements.

Those of skill in the art will appreciate that information and signals used to communicate the messages described herein may be represented using any of a variety of different technologies and techniques. For example, data, instructions, commands, information, signals, bits, symbols, and chips that may be referenced throughout the above description may be represented by voltages, currents, electromagnetic waves, magnetic fields or particles, optical fields or particles, or any combination thereof.

Terms, "and" and "or" as used herein, may include a variety of meanings that are also expected to depend at least in part upon the context in which such terms are used. Typically, "or" if used to associate a list, such as A, B, or C, is intended to mean A, B, and C, here used in the inclusive sense, as well as A, B, or C, here used in the exclusive sense. In addition, the term "one or more" as used herein may be used to describe any feature, structure, or characteristic in the singular or may be used to describe some combination of features, structures, or characteristics. However, it should be noted that this is merely an illustrative example and claimed subject matter is not limited to this example. Furthermore, the term "at least one of" if used to associate a list, such as A, B, or C, can be interpreted to mean A, B, C, or any combination of A, B, and/or C, such as AB, AC, BC, AA, ABC, AAB, AABBCCC, etc.

Further, while certain embodiments have been described using a particular combination of hardware and software, it should be recognized that other combinations of hardware and software are also possible. Certain embodiments may be implemented only in hardware, or only in software, or using combinations thereof. In one example, software may be implemented with a computer program product containing computer program code or instructions executable by one or more processors for performing any or all of the steps, operations, or processes described in this disclosure, where the computer program may be stored on a non-transitory computer readable medium. The various processes described herein can be implemented on the same processor or different processors in any combination.

Where devices, systems, components or modules are described as being configured to perform certain operations or functions, such configuration can be accomplished, for example, by designing electronic circuits to perform the operation, by programming programmable electronic circuits (such as microprocessors) to perform the operation such as by executing computer instructions or code, or processors or cores programmed to execute code or instructions stored on a non-transitory memory medium, or any combination thereof. Processes can communicate using a variety of techniques, including, but not limited to, conventional techniques for inter-process communications, and different pairs of processes may use different techniques, or the same pair of processes may use different techniques at different times.

In view of this description embodiments may include different combinations of features. Implementation examples are described in the following numbered clauses:
**Clause 1.** A system comprising: one or more processors in a first electronic device; and one or more processor-readable storage media storing instructions which, when executed by the one or more processors, cause performance of: (i) determining a first message format for a first message to be transmitted to a second electronic device over a communication channel between the first electronic device and the second electronic device, wherein: the first message is part of a sequence of messages communicated over the communication channel, the first message format is one of a plurality of message formats available for messages communicated over the communication channel, each message format in the plurality of message formats is associated with a corresponding level of security, at least one message format in the plurality of message formats requires a message to be encrypted, and the first message format allows the first message to be processed independently of other messages in the sequence of messages; (ii) generating the first message according to the first message format; and (iii) transmitting the first message to the second electronic device over the communication channel.
**Clause 2.** The system of clause 1, wherein each message format in the plurality of message formats is configured to carry a set of information that allows a message to be communicated at the corresponding level of security and to be processed independently irrespective of an order in which the message is received relative to an order in which the message is transmitted.
**Clause 3.** The system of clause 1 or 2, wherein when executed by the one or more processors, the instructions further cause performance of: establishing the communication channel through mutual authentication with the second electronic device, using a public key infrastructure of the system.
**Clause 4.** The system of any of clauses 1-3, wherein the first message is formatted as a digitally signed message or an unsigned message.
**Clause 5.** The system of clause 4, wherein the first message is formatted as a digitally signed and encrypted message, and wherein a second message in the sequence of messages is formatted as an unencrypted message.
**Clause 6.** The system of any of clauses 1-5, wherein generating the first message according to the first message format comprises: configuring a header of the first message to include an indication of the first message format.
**Clause 7.** The system of any of clauses 1-6, wherein generating the first message according to the first message format comprises: generating an ephemeral key pair including an ephemeral public key and an ephemeral private key; and encrypting a payload section of the first message, wherein the first message contains the ephemeral public key and is configured to be decrypted based on the ephemeral public key.
**Clause 8.** The system of clause 7, wherein: the payload section of the first message is encrypted using a symmetric key; the symmetric key is derivable using the ephemeral private key without the ephemeral public key; and the symmetric key is also derivable using the ephemeral public key without the ephemeral private key.
**Clause 9.** The system of clause 7 or 8, wherein when executed by the one or more processors, the instructions further cause performance of: deriving the symmetric key using the ephemeral private key in combination with a public key associated with the second electronic device.
**Clause 10.** The system of any of clauses 1-9, wherein the first electronic device, the second electronic device, or both the first electronic device and the second electronic device are medical devices.
**Clause 11.** A processor-implemented method comprising: (i) determining, by one or more processors of a first electronic device, a first message format for a first message to be transmitted to a second electronic device over a communication channel between the first electronic device and the second electronic device, wherein: the first message is part of a sequence of messages communicated over the communication channel, the first message format is one of a plurality of message formats available for messages communicated over the communication channel, each message format in the plurality of message formats is associated with a corresponding level of security, at least one message format in the plurality of message formats requires a message to be encrypted, and the first message format allows the first message to be processed independently of other messages in the sequence of messages; (ii) generating the first message according to the first message format; and (iii) transmitting the first message to the second electronic device over the communication channel.
**Clause 12.** The processor-implemented method of clause 11, wherein each message format in the plurality of message formats is configured to carry a set of information that allows a message to be communicated at the corresponding level of security and to be processed independently irrespective of an order in which the message is received relative to an order in which the message is transmitted.
**Clause 13.** The processor-implemented method of clause 11 or 12, further comprising: establishing the communication channel through mutual authentication with the second electronic device, using a public key infrastructure.
**Clause 14.** The processor-implemented method of any of clauses 11-13, wherein the first message is formatted as a digitally signed message or an unsigned message.
**Clause 15.** The processor-implemented method of clause 14, wherein the first message is formatted as a digitally signed and encrypted message, and wherein a second message in the sequence of messages is formatted as an unencrypted message.
**Clause 16.** The processor-implemented method of any of clauses 11-15, wherein generating the first message according to the first message format comprises: configuring a header of the first message to include an indication of the first message format.
**Clause 17.** The processor-implemented method of any of clauses 11-16, wherein generating the first message according to the first message format comprises: generating an ephemeral key pair including an ephemeral public key and an ephemeral private key; and encrypting a payload section of the first message, wherein the first message contains the ephemeral public key and is configured to be decrypted based on the ephemeral public key.
**Clause 18.** The processor-implemented method of clause 17, wherein: the payload section of the first message is encrypted using a symmetric key; the symmetric key is derivable using the ephemeral private key without the ephemeral public key; and the symmetric key is also derivable using the ephemeral public key without the ephemeral private key.
**Clause 19.** The processor-implemented method of clause 17 or 18, further comprising: deriving, by the one or more processors of the first electronic device, the symmetric key using the ephemeral private key in combination with a public key associated with the second electronic device.
**Clause 20.** One or more non-transitory processor-readable storage media storing instructions which, when executed by one or more processors of a first electronic device, cause performance of: (i) determining a first message format for a first message to be transmitted to a second electronic device over a communication channel between the first electronic device and the second electronic device, wherein: the first message is part of a sequence of messages communicated over the communication channel, the first message format is one of a plurality of message formats available for messages communicated over the communication channel, each message format in the plurality of message formats is associated with a corresponding level of security, at least one message format in the plurality of message formats requires a message to be encrypted, and the first message format allows the first message to be processed independently of other messages in the sequence of messages; (ii) generating the first message according to the first message format; and (iii) transmitting the first message to the second electronic device over the communication channel.

Further disclosed herein is the subject-matter of the following items:
1. A system comprising:
   one or more processors in a first electronic device; and
   one or more processor-readable storage media storing instructions which, when executed by the one or more processors, cause performance of:
      determining a first message format for a first message to be transmitted to a second electronic device over a communication channel between the first electronic device and the second electronic device, wherein:
         the first message is part of a sequence of messages communicated over the communication channel,
         the first message format is one of a plurality of message formats available for messages communicated over the communication channel,
         each message format in the plurality of message formats is associated with a corresponding level of security,
         at least one message format in the plurality of message formats requires a message to be encrypted, and
         the first message format allows the first message to be processed independently of other messages in the sequence of messages;
      generating the first message according to the first message format; and
      transmitting the first message to the second electronic device over the communication channel.
2. The system of item 1, wherein each message format in the plurality of message formats is configured to carry a set of information that allows a message to be communicated at the corresponding level of security and to be processed independently irrespective of an order in which the message is received relative to an order in which the message is transmitted.
3. The system of item 1 or 2, wherein when executed by the one or more processors, the instructions further cause performance of:
   establishing the communication channel through mutual authentication with the second electronic device, using a public key infrastructure of the system.
4. The system of item 1 or of one of items 1 to 3, wherein the first message is formatted as a digitally signed message or an unsigned message.
5. The system of item 4 or of one of items 1 to 4, wherein the first message is formatted as a digitally signed and encrypted message, and wherein a second message in the sequence of messages is formatted as an unencrypted message.
6. The system of item 1 or of one of items 1 to 5, wherein generating the first message according to the first message format comprises:
   configuring a header of the first message to include an indication of the first message format.
7. The system of item 1 or of one of items 1 to 6, wherein generating the first message according to the first message format comprises:
   generating an ephemeral key pair including an ephemeral public key and an ephemeral private key; and
   encrypting a payload section of the first message, wherein the first message contains the ephemeral public key and is configured to be decrypted based on the ephemeral public key.
8. The system of item 7, wherein:
   the payload section of the first message is encrypted using a symmetric key;
   the symmetric key is derivable using the ephemeral private key without the ephemeral public key; and
   the symmetric key is also derivable using the ephemeral public key without the ephemeral private key.
9. The system of item 8, wherein when executed by the one or more processors, the instructions further cause performance of:
   deriving the symmetric key using the ephemeral private key in combination with a public key associated with the second electronic device.
10. The system of item 1 or of one of items 1 to 9, wherein the first electronic device, the second electronic device, or both the first electronic device and the second electronic device are medical devices.
11. A processor-implemented method comprising:
   determining, by one or more processors of a first electronic device, a first message format for a first message to be transmitted to a second electronic device over a communication channel between the first electronic device and the second electronic device, wherein:
      the first message is part of a sequence of messages communicated over the communication channel,
      the first message format is one of a plurality of message formats available for messages communicated over the communication channel,
      each message format in the plurality of message formats is associated with a corresponding level of security,
      at least one message format in the plurality of message formats requires a message to be encrypted, and
      the first message format allows the first message to be processed independently of other messages in the sequence of messages;
   generating the first message according to the first message format; and
   transmitting the first message to the second electronic device over the communication channel.
12. The processor-implemented method of item 11, wherein each message format in the plurality of message formats is configured to carry a set of information that allows a message to be communicated at the corresponding level of security and to be processed independently irrespective of an order in which the message is received relative to an order in which the message is transmitted.
13. The processor-implemented method of item 11 or 12, further comprising:
   establishing the communication channel through mutual authentication with the second electronic device, using a public key infrastructure.
14. The processor-implemented method of item 11 or of one of items 11 to 13, wherein the first message is formatted as a digitally signed message or an unsigned message.
15. The processor-implemented method of item 14 or of one of items 11 to 14, wherein the first message is formatted as a digitally signed and encrypted message, and wherein a second message in the sequence of messages is formatted as an unencrypted message.
16. The processor-implemented method of item 11 or of one of items 11 to 15, wherein generating the first message according to the first message format comprises:
   configuring a header of the first message to include an indication of the first message format.
17. The processor-implemented method of item 11 or of one of items 11 to 16, wherein generating the first message according to the first message format comprises:
   generating an ephemeral key pair including an ephemeral public key and an ephemeral private key; and
   encrypting a payload section of the first message, wherein the first message contains the ephemeral public key and is configured to be decrypted based on the ephemeral public key.
18. The processor-implemented method of item 17, wherein:
   the payload section of the first message is encrypted using a symmetric key;
   the symmetric key is derivable using the ephemeral private key without the ephemeral public key; and
   the symmetric key is also derivable using the ephemeral public key without the ephemeral private key.
19. The processor-implemented method of item 18, further comprising:
   deriving, by the one or more processors of the first electronic device, the symmetric key using the ephemeral private key in combination with a public key associated with the second electronic device.
20. One or more non-transitory processor-readable storage media storing instructions which, when executed by one or more processors of a first electronic device, cause performance of:
   determining a first message format for a first message to be transmitted to a second electronic device over a communication channel between the first electronic device and the second electronic device, wherein:
      the first message is part of a sequence of messages communicated over the communication channel,
      the first message format is one of a plurality of message formats available for messages communicated over the communication channel,
      each message format in the plurality of message formats is associated with a corresponding level of security,
      at least one message format in the plurality of message formats requires a message to be encrypted, and
      the first message format allows the first message to be processed independently of other messages in the sequence of messages;
   generating the first message according to the first message format; and
   transmitting the first message to the second electronic device over the communication channel.

## Claims

1. A system comprising:
one or more processors in a first electronic device; and
one or more processor-readable storage media storing instructions which, when executed by the one or more processors, cause performance of:
determining a first message format for a first message to be transmitted to a second electronic device over a communication channel between the first electronic device and the second electronic device, wherein:
the first message is part of a sequence of messages communicated over the communication channel,
the first message format is one of a plurality of message formats available for messages communicated over the communication channel,
each message format in the plurality of message formats is associated with a corresponding level of security,
at least one message format in the plurality of message formats requires a message to be encrypted, and
the first message format allows the first message to be processed independently of other messages in the sequence of messages;
generating the first message according to the first message format; and
transmitting the first message to the second electronic device over the communication channel.

2. The system of claim 1, wherein each message format in the plurality of message formats is configured to carry a set of information that allows a message to be communicated at the corresponding level of security and to be processed independently irrespective of an order in which the message is received relative to an order in which the message is transmitted.

3. The system of claim 1 or 2, wherein when executed by the one or more processors, the instructions further cause performance of:
establishing the communication channel through mutual authentication with the second electronic device, using a public key infrastructure of the system.

4. The system of one of claims 1 to 3, wherein the first message is formatted as a digitally signed message or an unsigned message,
and/or
wherein the first message is formatted as a digitally signed and encrypted message, and wherein a second message in the sequence of messages is formatted as an unencrypted message.

5. The system of one of claims 1 to 4, wherein generating the first message according to the first message format comprises:
configuring a header of the first message to include an indication of the first message format.

6. The system of one of claims 1 to 5, wherein generating the first message according to the first message format comprises:
generating an ephemeral key pair including an ephemeral public key and an ephemeral private key; and
encrypting a payload section of the first message, wherein the first message contains the ephemeral public key and is configured to be decrypted based on the ephemeral public key.

7. The system of claim 6, wherein:
the payload section of the first message is encrypted using a symmetric key;
the symmetric key is derivable using the ephemeral private key without the ephemeral public key; and
the symmetric key is also derivable using the ephemeral public key without the ephemeral private key,
particularly wherein when executed by the one or more processors, the instructions further cause performance of:
deriving the symmetric key using the ephemeral private key in combination with a public key associated with the second electronic device.

8. The system of one of claims 1 to 7, wherein the first electronic device, the second electronic device, or both the first electronic device and the second electronic device are medical devices.

9. A processor-implemented method comprising:
determining, by one or more processors of a first electronic device, a first message format for a first message to be transmitted to a second electronic device over a communication channel between the first electronic device and the second electronic device, wherein:
the first message is part of a sequence of messages communicated over the communication channel,
the first message format is one of a plurality of message formats available for messages communicated over the communication channel,
each message format in the plurality of message formats is associated with a corresponding level of security,
at least one message format in the plurality of message formats requires a message to be encrypted, and
the first message format allows the first message to be processed independently of other messages in the sequence of messages;
generating the first message according to the first message format; and
transmitting the first message to the second electronic device over the communication channel.

10. The processor-implemented method of claim 9, wherein each message format in the plurality of message formats is configured to carry a set of information that allows a message to be communicated at the corresponding level of security and to be processed independently irrespective of an order in which the message is received relative to an order in which the message is transmitted.

11. The processor-implemented method of claim 9 or 10, further comprising:
establishing the communication channel through mutual authentication with the second electronic device, using a public key infrastructure.

12. The processor-implemented method of one of claims 9 to 11, wherein the first message is formatted as a digitally signed message or an unsigned message,
and/or
wherein the first message is formatted as a digitally signed and encrypted message, and wherein a second message in the sequence of messages is formatted as an unencrypted message.

13. The processor-implemented method of one of claims 9 to 12, wherein generating the first message according to the first message format comprises:
configuring a header of the first message to include an indication of the first message format.

14. The processor-implemented method of one of claims 9 to 13, wherein generating the first message according to the first message format comprises:
generating an ephemeral key pair including an ephemeral public key and an ephemeral private key; and
encrypting a payload section of the first message, wherein the first message contains the ephemeral public key and is configured to be decrypted based on the ephemeral public key, particularly wherein:
the payload section of the first message is encrypted using a symmetric key;
the symmetric key is derivable using the ephemeral private key without the ephemeral public key; and
the symmetric key is also derivable using the ephemeral public key without the ephemeral private key,
particularly further comprising:
deriving, by the one or more processors of the first electronic device, the symmetric key using the ephemeral private key in combination with a public key associated with the second electronic device.

15. One or more non-transitory processor-readable storage media storing instructions which, when executed by one or more processors of a first electronic device, cause performance of:
determining a first message format for a first message to be transmitted to a second electronic device over a communication channel between the first electronic device and the second electronic device, wherein:
the first message is part of a sequence of messages communicated over the communication channel,
the first message format is one of a plurality of message formats available for messages communicated over the communication channel,
each message format in the plurality of message formats is associated with a corresponding level of security,
at least one message format in the plurality of message formats requires a message to be encrypted, and
the first message format allows the first message to be processed independently of other messages in the sequence of messages;
generating the first message according to the first message format; and
transmitting the first message to the second electronic device over the communication channel.
